# NEW EUROPEAN PATENT SPECIFICATION

(11) **EP 1 771 180 B2**
(45) Date of publication and mention of the opposition decision: **05.01.2011**
(45) Mention of the grant of the patent: 31.10.2007
(21) Application number: 05763728.2
(22) Date of filing: 15.06.2005
(51) Int. Cl.: A61K 31/57, A61K 31/593, A61K 9/12, A61K 47/10, A61K 47/44, A61P 17/00, A61P 17/06, A61K 8/03, A61K 8/63, A61K 8/67, A61Q 17/04, A61Q 19/00, A61Q 19/08

(54) **COMPOSITION IN THE FORM OF A SPRAY COMPRISING A COMBINATION OF CLOBETASOL PROPIONATE AND CALCITRIOL, AN ALCOHOL PHASE AND AN OILY PHASE**
ZUSAMMENSETZUNG IN SPRAYFORM MIT EINER KOMBINATION AUS CLOBETASOLPROPIONAT UND CALCITRIOL, EINER ALKOHOLPHASE UND ÖLPHASE
COMPOSITION SOUS FORME DE SPRAY COMPRENANT UNE COMBINAISON DE PROPIONATE DE CLOBÉTASOL ET DE CALCITRIOL, UNE PHASE ALCOOLIQUE ET UNE PHASE HUILEUSE

(30) Priority: 17.06.2004 FR 0406616; 29.09.2004 US 951903
(43) Date of publication of application: 11.04.2007
(73) Proprietor: Galderma S.A., 6330 Cham (CH)
(72) Inventor: WILLCOX, Nathalie, FR-06460 Saint Vallier de Thiey (FR); ORSONI, Sandrine, F-06210 Mandelieu (FR)
(74) Representative: Allab, Myriam
(86) International application number: PCT/EP2005/007973
(87) International publication number: WO 2005/123091

(56) References cited:
- EP-A- 0 966 972
- WO-A-00/64450
- WO-A-2004/112798
- FR-A- 2 737 118
- FR-A- 2 738 745
- FR-A- 2 740 038
- US-A- 4 610 978
- US-A- 5 972 920
- US-A1- 2003 007 929
- US-A1- 2003 170 194
- LAMBA S ET AL: "Combination therapy with vitamin D analogues." BRITISH JOURNAL OF DERMATOLOGY, vol. 144, no. Supplement 58, April 2001 (2001-04), pages 27-32, XP002253887 ISSN: 0007-0963
- AUSTAD J ET AL: "Clobetasol propionate followed by calcipotriol is superior to calcipotriol alone in topical treatment of psoriasis." JOURNAL OF THE EUROPEAN ACADEMY OF DERMATOLOGY AND VENEREOLOGY: JEADV. NETHERLANDS JUL 1998, vol. 11, no. 1, July 1998 (1998-07), pages 19-24, XP001154650 ISSN: 0926-9959

## Description

The invention relates to an anhydrous composition in the form of a spray comprising a combination of clobetasol propionate and calcitriol as pharmaceutical active ingredient, an alcohol phase and an oily phase in a physiologically acceptable medium, to the process for its preparation and to its use in cosmetics and dermatology.

It is not conventional to use a combination of active principles in the treatment of dermatological complaints. The main difficulties encountered by those skilled in the art when combining two active principles are the problems of chemical instability and the interactions which the active principles may undergo when they are present in the same formulation.

Few treatments therefore exist which combine calcitriol and a corticoid. In fact, vitamin D and its derivatives are unstable in aqueous media and sensitive to acidic pH values, whereas corticoids, and more particularly clobetasol propionate, are sensitive to basic media. It was not therefore obvious to those skilled in the art to combine and stabilize an active ingredient of the vitamin D type and a corticosteroid in one and the same composition.

Calcitriol is a vitamin D analogue used to regulate the calcium level in the organism. Its use in the treatment of dermatological diseases has been described especially in patent US 4,610,978 for the treatment of psoriasis. Said patent suggests compositions comprising calcitriol that can also contain an amount of an anti-inflammatory such as a corticosteroid, but no concrete embodiment of a combination of calcitriol and a corticosteroid is either described or tested in terms of efficacy.

In patent application FR 2 848 454 the Applicant described that a combination of calcitriol with a corticosteroid made it possible to obtain a synergistic effect in the treatment of certain dermatological complaints such as psoriasis, atopic dermatitis, contact dermatitis and seborrhoeic dermatitis, without however proposing stable pharmaceutical compositions combining both active ingredients.

Furthermore, in the field of dermatology and the formulation of pharmaceutical compositions, those skilled in the art are induced to look for compositions which not only have to be physically and chemically stable, but also have to make it possible to release the active ingredient and promote its penetration through the cutaneous layers so as to improve its efficacy.

The pharmaceutical compositions moreover have to have a good cosmetic character and preferably be non-irritant.

There are currently numerous topical compositions that comprise an active ingredient and are capable of promoting its penetration into the skin by virtue of the presence especially of a high content of propenetrating glycol. These compositions are formulated as emulsions with a high content of fatty phase, commonly called "lipocreams", as anhydrous compositions called "unguents", as fluid compositions with a high content of volatile solvents such as ethanol or isopropanol, intended for application to the scalp and also called "hair lotions", or as viscous O/W emulsions, also called "O/W creams".

The stabilization of a formulation comprising such a percentage of glycol makes it necessary to use, in the emulsion, emulsifiers and stabilizers of the glyceryl stearate or PEG 100 stearate type, or stabilizers or consistency factors of the white wax or cetostearyl alcohol type, which give rise to the formation of a viscous cream, i.e. a cream with a viscosity greater than 10 Pa.s (10,000 centipoises, measured with a Brookfield LVDV II apparatus + no. 4 cup, at a speed of 30 rpm for 30 seconds and at a temperature of 25°C ± 3°C). This viscosity therefore makes the product difficult to apply. Hence these compositions on the one hand have a poor cosmetic acceptability due to their viscosity, and on the other hand carry risks of intolerance caused by the presence of high proportions of glycol. In addition, these high viscosities make the formulations difficult to apply to the different parts of the body affected by the pathological condition. Consequently, the majority of existing treatments, in the form of creams, gels or ointments, require the help of a third party to apply them to the areas that are difficult to reach. The third party therefore has to touch both the product containing the active ingredient and the psoriatic plaques, resulting in a situation that is not ideal from the point of view of the comfort of the user and the safety of the third party. Those skilled in the art are also aware that non-compliance with the prescribed treatment for reasons referred to above is one of the main causes of failure, the article *"*Patients with psoriasis and their compliance with medication" (Richards et al., J. Am. Acad. Dermatol., Oct. 99, pp 581-583) indicating that nearly 40% of patients with a chronic disease like psoriasis do not follow their treatment. It has been demonstrated that the patient's compliance with his treatment is directly related to the characteristics of the vehicle of the composition applied. The article *"*Patients with psoriasis prefer solution and foam vehicles: a quantitative assessment of vehicle preference" (Housman et al., CUTIS, Dec. 2002, vol. 70, pp 327 to 332) indicates that psoriasis patients prefer a solution or a foam to an unguent, a cream or a gel.

It thus appears desirable to improve the comfort on use of this type of composition, which is what the Applicant has succeeded in doing in the present invention.

The prior art closest to the invention is international patent application WO 00/64450, which mentions the use of a pharmaceutical composition containing a vitamin D analogue and a corticosteroid. All the composition examples in said patent application combine solely calcipotriol and betamethasone dipropionate. The preferred compositions described in the patent application that make it possible to stabilize the two active ingredients are compositions in the form of an unguent. However, these compositions exhibit the abovementioned disadvantages as regards comfort and ease of application. Reading this prior art in no way enables those skilled in the art to infer sprayable, i.e. easily applicable, compositions such as those described in the present patent application with the active ingredients clobetasol propionate and calcitriol, which are solubilized and stable in the composition.

US patent 2003/007929 on the other hand describes a topical spray comprising clobetasol propionate, an alcohol, isopropyl myristate and a propellant. This prior art however does not disclose a sprayable composition comprising both clobetasol propionate and calcitriol.

The problem which the present invention sets out to solve here is therefore to devise a physically and chemically stable composition that allows the two active ingredients calcitriol and clobetasol propionate to be combined in one and the same composition, said ingredients acting synergistically for the treatment of psoriasis, the composition according to the invention also being easy to use and having an acceptable cosmetic character for application to all areas of the body that may be affected by the pathological condition.

"Physical stability" is understood according to the invention as applying to a composition that does not undergo any modification of macroscopic appearance (phase separation, change of colour or appearance, etc.) or microscopic appearance (recrystallization of active ingredients) after storage at temperatures of 4°C and 40°C for 2, 4, 8 and 12 weeks.

"Chemical stability" is understood according to the invention as applying to a composition in which the active principle content remains stable after three months at room temperature and at 40°C. A stable active principle content means according to the invention that the content varies very little relative to the initial content, i.e. that the variation in active principle content at time T must not be less than 90% of the initial content at T0 and preferably not less than 95% of the initial content at T0.

The Applicant has found, surprisingly, that a composition liquid at room temperature and sprayable comprising the following in a pharmaceutically acceptable vehicle:
a) between 0.0001 and 0.1% of clobetasol propionate in solubilized form;
b) between 0.00001 and 0.1% of calcitriol in solubilized form;
c) between 30 and 60% of ethanol;
d) between 5 and 90% of an oily phase composed of one or more oils chosen from caprylic/capric triglycerides, cetearyl isononanoate and vegetable oils.
solves the problems posed.

While allowing a good penetration of the active principles, the composition of the present invention is chemically and physically stable. It also has a very good patient acceptability and tolerance, due to its spray formula, as described below in the examples of the present invention. The composition according to the invention is therefore found to be particularly suitable for the treatment of dermatological complaints and more particularly for the treatment of psoriasis.

The invention therefore relates to a composition liquid at room temperature and sprayable, comprising the following in a pharmaceutically acceptable vehicle:
a) between 0.0001 and 0.1% of clobetasol propionate in solubilized form;
b) between 0.00001 and 0.1% of calcitriol in solubilized form;
c) between 30 and 60% of ethanol;
d) between 5 and 90% of an oily phase composed of one or more oils chosen from caprylic/capric triglycerides, cetearyl isononanoate and vegetable oils.

"Solubilized form" is understood as applying to a dispersion in the molecular state in a liquid, no crystallization of the active ingredient being visible to the naked eye nor even under a cross-polarizing optical microscope.

"Sprayable composition" is understood as applying a fluid liquid composition that flows rapidly under its own weight, at room temperature. "Room temperature" is understood as applying to a temperature of approximately 25°C.

The spray can be obtained by conventional formulation means known to those skilled in the art, as is explained hereinafter.

Preferably, the composition is anhydrous. For the purpose of the present invention, "anhydrous composition" is understood as applying to a composition substantially free of water, i.e. having a water content of less than or equal to 1% by weight relative to the total weight of the composition, in particular less than or equal to 0.5%, preferably equal to zero.

The composition according to the invention comprises between 0.00001 and 0.1% by weight, preferably between 0.0001 and 0.001% by weight and particularly preferably between 0.0002 and 0.0005% by weight of calcitriol, based on the total weight of the composition. The composition according to the invention comprises more particularly 0.0003% by weight of calcitriol, based on the total weight of the composition.

The composition according to the invention comprises between 0.0001 and 0.1% by weight and preferably between 0.001 and 0.05% by weight of clobetasol propionate, based on the total weight of the composition. The preferred compositions according to the invention comprise more particularly 0.01%, 0.025% or 0.05% by weight of clobetasol propionate, based on the total weight of the composition.

"Alcohol phase" is understood according to the invention as meaning at least one alcohol compound. Examples which may be mentioned of alcoholic compounds usable according to the invention are linear or branched aliphatic alcohols such as anhydrous or non-anhydrous ethanol; isopropanol and butanol. The composition according to the invention contains ethanol between 30 and 60% by weight and preferably between 35 and 45% by weight of ethanol based on the total weight of the composition.

A preferred composition according to the invention contains between 35 and 45% by weight of ethanol.

"Oily phase" is understood according to the invention as meaning an oily phase that is appropriate for a pharmaceutical or cosmetic composition. Oils generally have a viscosity above about 10 centipoises at 25°C and can reach a viscosity ranging up to 1 000 000 centipoises at 25°C.

The oily phase of the composition according to the invention is composed of one or more oils chosen from the caprylic/capric triglycerides marketed under the name Miglyol 812, the cetearyl isononanoate marketed under the name Cetiol SN, and vegetable oils (sweet-almond oil, sesame oil, wheatgerm oil, olive oil, jojoba oil, etc.).

The composition according to the invention comprises between 5 and 90% by weight, preferably between 20 and 80% by weight and particularly preferably between 50 and 70% by weight of oily phase, based on the total weight.

The composition according to the invention thus comprises, in a pharmaceutically acceptable vehicle:
a) between 0.0001 and 0.1% of clobetasol propionate in solubilized form;
b) between 0.00001 and 0.1% of calcitriol in solubilized form;
c) between 30 and 60% of ethanol;
d) between 5 and 90% of an oily phase composed of one or more oils chosen from caprylic/capric triglycerides, cetearyl isononanoate and vegetable oils.

Preferably, the composition according to the invention thus comprises, in a pharmaceutically acceptable vehicle:
a) between 0.001 and 0.05% of clobetasol propionate;
b) between 0.0002 and 0.0005% of calcitriol;
c) between 35 and 45% of ethanol;
d) between 20 and 80% of an oily phase composed of one or more oils chosen from caprylic/capric triglycerides, cetearyl isononanoate and vegetable oils.

In one preferred embodiment, the composition according to the invention also contains antioxidant compounds such as DL-α-tocopherol, butylhydroxyanisole or butylhydroxytoluene, propyl gallate, superoxide dismutase, ubiquinol or certain metal chelating agents. The antioxidants preferably used in the composition according to the invention are DL-α-tocopherol, butylhydroxyanisole and butylhydroxytoluene.

The composition according to the invention can also contain surfactants. Such compositions can in fact provide a moderate keratolytic action and can help maintain the solubilization of the active ingredients. The surfactants usable according to the invention are of the anionic surfactant type such as carboxylates and especially soaps, alkylarylsulphonates, alkylethersulphates, alkylsulphates and alcohol sulphates. More particularly, the anions of these surfactants are coupled with a cation such as that of the metal sodium or potassium. Other preferred surfactants according to the invention are those of the polysorbate and poloxamer types.

Preferably, the surfactants used according to the present invention are sodium laurylsulphate, polysorbate 80 (TWEEN 80 from Uniqema) and poloxamer 124 (SYNPERONIC PEL44 from Uniqema).

The pharmaceutical composition according to the invention may also contain inert additives or combinations of these additives, such as
- wetting agents;
- flavour improvers;
- preservatives;
- stabilizers;
- humidity regulators;
- pH regulators;
- osmotic pressure modifiers;
- emulsifiers;
- UV-A and UV-B filters;
- propenetrating agents; and
- synthetic polymers.

Of course, those skilled in the art will take care to choose any compound(s) to be added to these compositions in such a way that the advantageous properties intrinsically associated with the present invention are unaffected or substantially unaffected by the envisaged addition.

The composition according to the invention is more particularly intended for the treatment of skin and mucosae; it is sprayable and suitable for packaging in the form of a spray.

The spray has numerous advantages compared with conventional forms, such as easy delivery of the formula to the areas of the body which are very difficult to treat, possible simple control of the dose delivered or the absence of contamination during use.

The composition according to the invention is therefore administered in the form of a sprayable composition. The latter can be obtained by conventional formulating means known to those skilled in the art. For example, the composition can be sprayed by a mechanical sprayer which pumps the composition from a container, bottle or equivalent vessel. Likewise, the composition can be propelled by means of a gas in the manner well known to those skilled in the art. The conventional propellant gases, such as air or hydrocarbons, are effective provided they do not interfere with the composition. The composition passes through a nozzle, which can be pointed directly at the desired application site. The nozzle can be chosen so as to apply the composition in the form of a vapour or a jet of droplets according to the techniques known to those skilled in the art. Depending on the chosen pharmaceutical active ingredient, the spraying mechanism must be capable always of dispensing the same amount of active ingredient. The mechanisms for controlling the amount of composition to be dispensed by the spray are also known to those skilled in the art. For example, the amount of propellant gas can be calculated so as to propel the exact amount of product desired. For the composition according to the invention, it is possible to use a dosing vaporizer bottle whose characteristics of application area and dose are controlled and reproducible. For example, the vaporizer can consist of a bottle equipped with a dosing valve.

While allowing a good penetration of the active principles, the composition of the present invention is chemically and physically stable. It also has a very good patient acceptability and tolerance, due to its spray formula, as described in the examples of the present invention. The composition according to the invention is therefore found to be particularly suitable for the treatment of dermatological complaints.

The present invention therefore further relates to the use of a composition according to the invention for the preparation of a drug intended for the treatment of:
- dermatological complaints with an inflammatory immunoallergic component and with or without cellular proliferation disorder, especially cutaneous, mucous or ungueal psoriasis, psoriatic rheumatism, and cutaneous atopy such as eczema.

In a preferred mode of use of the composition according to the invention, it will be used for the preparation of a drug intended to treat psoriasis.

In particular, the compositions as defined above comprise 0.01%, 0.025% or 0.05% of clobetasol 17-propionate and 0.0003% of calcitriol in the presence of ethanol.

The examples which follow are a non-exhaustive representation of formulation examples of the composition according to the invention, together with chemical and physical stability results and results of the test of release-penetration of the active ingredients.

### Example 1: Stability of calcitriol in various excipients

The following example describes the calcitriol stability data in various excipients, including ethanol 100, caprylic/capric triglycerides and cetearyl isononanoate, preferred excipients for the composition according to the invention.

### a) Stability of calcitriol in ethanol

Solution of 30 ppm of calcitriol in qsp 100% of absolute ethanol, in the presence of 0.02% of BHT.

Technique of HPLC assay against a reference substance.

At the starting time (T0) the composition is considered to comprise 100% of calcitriol.

Measured concentration of calcitriol in % relative to T0:

| Stability conditions | T 1 week | T 2 weeks | T 3 weeks | T 4 weeks |
|---|---|---|---|---|
| -18°C | 100.9% | 100.5% | 99.5% | 99.5% |
| +4°C | 97.7% | 98.6% | 98.1% | 97.7% |
| +30°C | / | 93.4% | / | 93.0% |

### b) Stability of calcitriol in Miglyol 812 (caprylic/capric triglycerides)

Solution of 30 ppm of calcitriol in qsp 100% of Miglyol 812, in the presence of 0.4% of BHT.

Technique of HPLC assay against a reference substance.

At the starting time (T0) the composition is considered to comprise 100% of calcitriol.

Measured concentration of calcitriol in % relative to T0:

| Stability conditions | T 2 weeks | T 4 weeks |
|---|---|---|
| +4°C | 98.3% | 105.2% |
| RT | 95.1% | 98.0% |
| +40°C | 91% | 93.8% |

### c) Stability of calcitriol in Cetiol SN (cetearyl isononanoate)

Solution of 30 ppm of calcitriol in qsp 100% of Cetiol SN (cetearyl isononanoate), in the presence of 0.4% of BHT.

Technique of HPLC assay against a reference substance.

At the starting time (T0) the composition is considered to comprise 100% of calcitriol.

Measured concentration of calcitriol in % relative to T0:

| Stability conditions | T 2 weeks | T 4 weeks |
|---|---|---|
| +4°C | 98.6% | 98.1% |
| RT | 98.7% | 98.4% |
| +40°C | 99.0% | 98.9% |

### Example 2: Process for the preparation of the compositions according to the invention

The compositions according to the invention are prepared at room temperature, under a hood and in inactinic light.

The antioxidant, the calcitriol and the alcohol are introduced into a flask and stirred until the calcitriol is perfectly solubilized.

The clobetasol propionate is then added and stirring is continued until the clobetasol propionate is solubilized.

When the two active ingredients are perfectly solubilized, the remaining constituents of the formulation are introduced in succession.

The mixture is stirred until it is perfectly homogeneous.

### Reference Example 3

| CONSTITUENTS | % |
|---|---|
| 2-PROPANOL | qs 100 |
| DL-ALPHA-TOCOPHEROL ACETATE | 0.04 |
| CALCITRIOL | 0.0003 |
| CLOBETASOL 17-PROPIONATE | 0.001 |
| SESAME OIL | 5 |
| MEDIUM CHAIN TRIGLYCERIDES | 55 |
| POLOXAMER 124 | 0.10 |

The procedure is the one described in Example 2.

A slightly yellow liquid solution is obtained.

### Example 4

| CONSTITUENTS | % |
|---|---|
| ABSOLUTE ETHANOL | qs 100 |
| BUTYLHYDROXYTOLUENE | 0.04 |
| CALCITRIOL | 0.0003 |
| CLOBETASOL 17-PROPIONATE | 0.025 |
| ALMOND OIL | 5 |
| MEDIUM CHAIN TRIGLYCERIDES | 55 |
| POLYSORBATE 80 | 0.10 |

The procedure is the one described in Example 2.

A slightly yellow liquid solution is obtained.

### Example 5

| CONSTITUENTS | % |
|---|---|
| ABSOLUTE ETHANOL | qs 100 |
| BUTYLHYDROXYTOLUENE | 0.04 |
| CALCITRIOL | 0.0003 |
| CLOBETASOL 17-PROPIONATE | 0.025 |
| 1,2-PROPANEDIOL | 10 |
| MEDIUM CHAIN TRIGLYCERIDES | 35 |
| ALMOND OIL | 5 |
| POLYSORBATE 80 | 0.10 |

The procedure is the one described in Example 2.

A slightly yellow liquid solution is obtained.

### Example 6

| CONSTITUENTS | % |
|---|---|
| ABSOLUTE ETHANOL | qs 100 |
| BUTYLHYDROXYTOLUENE | 0.04 |
| CALCITRIOL | 0.0003 |
| CLOBETASOL 17-PROPIONATE | 0.025 |
| 1,2-PROPANEDIOL | 10 |
| MEDIUM CHAIN TRIGLYCERIDES | 40 |
| POLYSORBATE 80 | 0.10 |

The procedure is the one described in Example 2.

A colourless liquid solution is obtained.

### Example 7 - physical stability of the composition according to Example 6

The physical stability of the formulations is measured by macroscopic and microscopic observation of the formulation at room temperature, at 4°C and at 40°C after 2, 4, 8 and 12 weeks.

At room temperature, macroscopic observation makes it possible to guarantee the physical integrity of the products and microscopic observation makes it possible to verify that there is no recrystallization of the solubilized active ingredient.

Non-recrystallization of the solubilized active ingredients is verified by microscopic observation at 4°C.

The integrity of the finished product is verified by macroscopic observation at 40°C.

### Specifications at T0

*Macroscopic appearance:* colourless liquid spray

*Microscopic appearance:* absence of crystals of calcitriol and clobetasol 17-propionate

| Time→ Stability conditions↓ | T 1M | T2M |
|---|---|---|
| RT | conforms to the specification | conforms to the specification |
| +4°C | conforms to the specification | conforms to the specification |
| +40°C | conforms to the specification | conforms to the specification |

### Example 8 - chemical stability of the active ingredients within the composition according to Example 6

### Stability of the calcitriol

Assay of the active ingredient is carried out by external calibration using HPLC.

| Time→ Stability conditions↓ | T 0 | T 15d | T 1M | T2M |
|---|---|---|---|---|
| RT | 96.6% | 95.4% | 93.6% | 94.5% |
| +40°C | / | 93.6% | 90.8% | 92.8% |

### Stability of the clobetasol 17-propionate

### Assay of the active ingredient by internal calibration using HPLC.

| Time→ Stability conditions↓ | T 0 | T 15d | T 1M | T2M |
|---|---|---|---|---|
| RT | 97.4% | 95.9% | 98.8% | 97.7% |
| +40°C | / | 95.5% | 98.2% | 97.2% |

### Example 9

| CONSTITUENTS | % |
|---|---|
| ABSOLUTE ETHANOL | qs 100 |
| BUTYLHYDROXYTOLUENE | 0.04 |
| CALCITRIOL | 0.0003 |
| CLOBETASOL 17-PROPIONATE | 0.025 |
| MEDIUM CHAIN TRIGLYCERIDES | 40 |
| POLYSORBATE 80 | 0.10 |

The procedure is the one described in Example 2.

The composition obtained is a clear liquid solution.

### Example 10: physical stability of the composition according to Example 9

| Time→ Stability conditions↓ | T 1M | T2M | T3M |
|---|---|---|---|
| RT | Conforms to the specification | Conforms to the specification | Conforms to the specification |
| +4°C | Conforms to the specifi**c**ation | Conforms to the specification | Conforms to the specification |
| +40°C | Conforms to the specification | Conforms to the specification | Conforms to the specification |

### Example 11: chemical stability of the active ingredients within the composition according to Example 9

### Stability of the calcitriol

Assay of the active ingredient is carried out by external calibration using HPLC.

| Time→ Stability conditions↓ | T 0 | T 1M | T2M | T3M |
|---|---|---|---|---|
| RT | 101.1% | 96% | 91.4% | 103.5% |
| +40°C | / | 102.9% | 100.2% | 103.1% |

### Stability of the clobetasol 17-propionate

Assay of the active ingredient by internal calibration using HPLC.

| Time→ Stability conditions↓ | T 0 | T 1M | T2M | T3M |
|---|---|---|---|---|
| RT | 98.3% | 100% | 99.6% | 100.1% |
| +40°C | / | 99% | 98.8% | 99.2% |

### Example 12

| CONSTITUENTS | % |
|---|---|
| ABSOLUTE ETHANOL | qs 100 |
| BUTYLHYDROXYTOLUENE | 0.04 |
| CALCITRIOL | 0.0003 |
| CLOBETASOL 17-PROPIONATE | 0.05 |
| MEDIUM CHAIN TRIGLYCERIDES | 60 |
| POLYSORBATE 80 | 0.10 |

The procedure is the one described in Example 2.

A clear liquid solution is obtained.

### Example 13: physical stability of the composition according to Example 12

The stability of the formulations is measured by macroscopic and microscopic observation of the formulation at room temperature, at 4°C and at 40°C after 2, 4, 8 and 12 weeks.

At room temperature, macroscopic observation makes it possible to guarantee the physical integrity of the products and microscopic observation makes it possible to verify that there is no recrystallization of the solubilized active ingredient.

Non-recrystallization of the solubilized active ingredients is verified by microscopic observation at 4°C.

The integrity of the finished product is verified by macroscopic observation at 40°C.

### Specifications at T0

*Macroscopic appearance:* colourless liquid spray.

*Microscopic appearance:* absence of crystals of calcitriol and of clobetasol 17-propionate.

| Time→ Stability conditions↓ | T 1M | T2M | T3M |
|---|---|---|---|
| RT | Conforms to the specification | Conforms to the specification | Conforms to the specification |
| +4°C | Conforms to the specification | Conforms to the specification | Conforms to the specification |
| +40°C | Conforms to the specification | Conforms to the specification | Conforms to the specification |

### Example 14: chemical stability of the active ingredients within the composition according to Example 12

| *Stability of the calcitriol* | | | |
|---|---|---|---|
| Time→ Stability conditions↓ | T 1M | T2M | T3M |
| RT | 96.7% | 97% | 100% |
| +40°C | 97.8% | 98.4% | 100.9% |

| *Stability of the clobetasol 17-propionate* | | | |
|---|---|---|---|
| Time→ Stability conditions↓ | T 1M | T2M | T3M |
| RT | 99.4% | 95.8% | 99.7% |
| +40°C | 99.6% | 96% | 99.5% |

### Example 15

| CONSTITUENTS | % |
|---|---|
| ABSOLUTE ETHANOL | qs 100 |
| BUTYLHYDROXYTOLUENE | 0.04 |
| CALCITRIOL | 0.0003 |
| CLOBETASOL 17-PROPIONATE | 0.025 |
| MEDIUM CHAIN TRIGLYCERIDES | 60 |
| POLYSORBATE 80 | 0.10 |

The procedure is the one described in Example 2.

A clear liquid solution is obtained.

### Example 16: physical stability of the composition according to Example 15

The stability of the formulations is measured by macroscopic and microscopic observation of the formulation at room temperature, at 4°C and at 40°C after 2, 4, 8 and 12 weeks.

At room temperature, macroscopic observation makes it possible to guarantee the physical integrity of the products and microscopic observation makes it possible to verify that there is no recrystallization of the solubilized active ingredient.

Non-recrystallization of the solubilized active ingredients is verified by microscopic observation at 4°C.

The integrity of the finished product is verified by macroscopic observation at 40°C.

### Specifications at T0

*Macroscopic* appearance: colourless liquid spray.

*Microscopic appearance:* absence of crystals of calcitriol and of clobetasol 17-propionate.

| Time→ Stability conditions↓ | T 1M | T2M | T3M |
|---|---|---|---|
| RT | Conforms to the specification | Conforms to the specification | Conforms to the specification |
| +4°C | Conforms to the specification | Conforms to the specification | Conforms to the specification |
| +40°C | Conforms to the specification | Conforms to the specification | Conforms to the specification |

### Example 17: chemical stability of the active ingredients within the composition according to Example 15

| *Stability of the calcitriol* | | | |
|---|---|---|---|
| Time→ Stability conditions↓ | T 1M | T2M | T3M |
| RT | 96.6% | 97.3% | 99.3% |
| +40°C | 96.9% | 97.6% | 99.8% |

| *Stability of the clobetasol 17-propionate* | | | |
|---|---|---|---|
| Time→ Stability conditions↓ | T 1M | T2M | T3M |
| RT | 98.8% | 96% | 99.7% |
| +40°C | 99.1% | 96.1% | 99.3% |

### Reference Example 18: Study of the release/penetration, in vitro, on human skin of the active ingredient clobetasol 17-propionate contained in 3 different formulations

The objective is to quantify the penetration into the skin of the active ingredient formulated in various formulations, *in vitro,* on human skin after 16 hours of application.

### Formulations tested:

- Temovate® emollient cream containing 0.05% (w/w) of clobetasol 17-propionate
- Temovate® cream containing 0.05% (w/w) of clobetasol 17-propionate
- Composition according to the invention of formula A below:

| CONSTITUENTS | % |
|---|---|
| 2-PROPANOL | qs 100 |
| DL ALPHA TOCOPHEROL ACETATE | 0.04 |
| CALCITRIOL | 0.0003 |
| CLOBETASOL 17-PROPIONATE | 0.05 |
| SESAME OIL | 5 |
| MEDIUM CHAIN TRIGLYCERIDES | 55 |
| POLOXAMER 124 | 0.10 |

The Temovate® emollient cream is sold by the company GlaxoSmithKline.

**Experimental conditions**: The percutaneous absorption is evaluated using diffusion cells consisting of two compartments separated by human skin. The formulations were applied without occlusion for an application time of 16 hours. The formulations were applied in a proportion of 10 mg of formulation per cm² (i.e. 10 micrograms of clobetasol 17-propionate). Throughout the study, the dermis is in contact with a recipient liquid that is not renewed as a function of time (static mode). The experiments were carried out with 3 skin samples originating from 3 different donors. At the end of the application period, the surface excess is removed and the distribution of clobetasol 17-propionate is quantified in the various compartments of the skin and in the recipient liquid. The concentrations of clobetasol 17-propionate were quantified using an HPLC/MS/MS method conventionally known to those skilled in the art (LQ: 1 ng.mL⁻¹).

The results are expressed as % of the dose applied (mean +/- standard deviation) and are given in the table below.

| Formulation | | **Total amount having penetrated** |
|---|---|---|
| Temovate emollient cream | Mean | **5.00** |
| | SEM | **1.34** |
| Temovate cream | Mean | **8.43** |
| | SEM | **0.79** |
| Composition A | Mean | **4.96** |
| | SEM | **0.69** |

The results show that the amount of clobetasol that has penetrated with the composition according to the invention is equivalent to that of the Temovate emollient cream.

## Claims

1. Composition comprising the following in a pharmaceutically acceptable vehicule:
a) between 0.0001 and 0.1% of clobetasol propionate in solubilized form;
b) between 0.00001 and 0.1% of calcitriol in solubilized form;
c) between 30 and 60% of ethanol;
d) between 5 and 90% of an oily phase composed of one or more oils selected from caprylic/capric triglycerides, cetearyl isononanoate and vegetable oils,
**characterized in that** the composition is liquid at room temperature and sprayable.

2. Composition according to claim 1 comprising the following in a pharmaceutically acceptable vehicule:
a) between 0.001 and 0.05% of clobetasol propionate;
b) between 0.0002 and 0.0005% of calcitriol;
c) between 35 and 45% of ethanol;
d) between 20 and 80% of an oily phase composed of one or more oils elected from caprylic/capric triglycerides, cetearyl isononanoate and vegetable oils.

3. Composition according to any one of claims 1 to 2, **characterized in that** it also comprises an antioxidant compound.

4. Composition according to claim 3, **characterized in that** the antioxidant is selected from DL-α-tocopherol, butylhydroxyanisole and butylhydroxytoluene.

5. Composition according to any one of claims 1 to 4, **characterized in that** if also comprises a surfactant compound.

6. Composition according to claim 5, **characterized in that** the surfactant is selected from sodium laurylsulphate, poloxamers and polysorbates.

7. Composition according to any one of claims 1 to 6, **characterized in that** it comprises a propenetrating agent.

8. Use of a composition according to any one of claims 1 to 7, for the preparation of a drug intended for the treatment of:
- dermatological complaints with an inflammatory immunoallergic component and with or without cellular proliferation disorder, especially cutaneous, mucous or ungueal psoriasis, psoriatic rheumatism, and cutaneous atopy such as eczema.

9. Use according to claim 8, for the treatment of psoriasis.

## Patentansprüche

1. Zusammensetzung, die in einem pharmazeutisch akzeptablen Träger enthält:
a) 0,0001 bis 0,1 % Clobetasolpropionat in solubilisierter Form;
b) 0,00001 bis 0,1 % Calcitriol in solubilisierter Form;
c) 30 bis 60 % Ethanol;
d) 5 bis 90 % Ölphase, die aus einem oder mehreren Ölen zusammengesetzt ist, die unter Capryl/ Caprinsäuretriglyceriden, Cetearylisononanoat und pflanzlichen Ölen ausgewählt sind.
**dadurch gekennzeichnet, dass** die Zusammensetzung bei Raumtemperatur flüssig und versprühbar ist.

2. Zusammensetzung nach Anspruch 1, die in einem pharmazeutisch akzeptablen Träger enthält:
a) 0,001 bis 0,05 % Clobetasolpropionat;
b) 0,0002 bis 0,0005 % Calcitriol;
c) 35 bis 45 % Ethanol;
d) 20 bis 80 % Ölphase, die aus einem oder mehreren Ölen besteht, die unter Capryl/Caprinsäuretriglyceriden, Cetearylisononanoat und pflanzlichen Ölen ausgewählt sind.

3. Zusammensetzung nach einem der Ansprüche 1 bis 2, **dadurch gekennzeichnet, dass** sie ferner ein Antioxidationsmittel enthält.

4. Zusammensetzung nach Anspruch 3, **dadurch gekennzeichnet, dass** das Antioxidationsmittel unter DL-α-Tocopherol, Butylhydroxyanisol und Butylhydroxytoluol ausgewählt ist.

5. Zusammensetzung nach einem der Ansprüche 1 bis 4, **dadurch gekennzeichnet, dass** sie auch einen grenzflächenaktiven Stoff enthält.

6. Zusammensetzung nach Anspruch 5, **dadurch gekennzeichnet, dass** der grenzflächenaktive Stoff unter Natriumlaurylsulfat, Poloxameren und Polysorbaten ausgewählt ist.

7. Zusammensetzung nach einem der Ansprüche 1 bis 6, **dadurch gekennzeichnet, dass** sie einen penetrationsfördernden Stoff enthält.

8. Verwendung einer Zusammensetzung nach einem der Ansprüche 1 bis 7 für die Herstellung eines Arzneimittels zur Behandlung von:
- dermatologischen Erkrankungen mit einer entzündlichen immunoallergischen Komponente und mit oder ohne Störungen der Zellproliferation, insbesondere Psoriasis der Haut, Psoriasis der Schleimhaut oder Psoriasis des Nagels, Psoriasis-Arthritis, Atopie der Haut, wie Ekzemen.

9. Verwendung nach Anspruch 8 für die Behandlung von Psoriasis.

## Revendications

1. Composition comprenant les éléments suivants dans un véhicule pharmaceutiquement acceptable :
a) entre 0,0001 et 0,1 % de propionate de clobétasol sous forme solubilisée ;
b) entre 0,00001 et 0,1 % de calcitriol sous forme solubilisée ;
c) entre 30 et 60 % d'éthanol ;
d) entre 5 et 90 % d'une phase huileuse composée d'une ou de plusieurs huiles choisies parmi les triglycérides capryliques/capriques, l'isononanoate de cétéaryle et les huiles végétales,
**caractérisée en ce que** la composition est liquide à température ambiante et pulvérisable.

2. Composition selon la revendication 1, comprenant les éléments suivants dans un véhicule pharmaceutiquement acceptable :
a) entre 0,001 et 0,05 % de propionate de clobétasol ;
b) entre 0,0002 et 0,0005 % de calcitriol ;
c) entre 35 et 45 % d'éthanol ;
d) entre 20 et 80 % d'une phase huileuse composée d'une ou de plusieurs huiles choisies parmi les triglycérides capryliques/capriques, l'isononanoate de cétéaryle et les huiles végétales.

3. Composition selon l'une quelconque des revendications 1 à 2, **caractérisée en ce qu'**elle comprend également un composé antioxydant.

4. Composition selon la revendication 3, **caractérisée en ce que** l'antioxydant est choisi parmi le DL-α-tocophérol, le butylhydroxyanisole et le butylhydroxytoluène.

5. Composition selon l'une quelconque des revendications 1 à 4, **caractérisée en ce qu'**elle comprend également un composé tensioactif.

6. Composition selon la revendication 5, **caractérisée en ce que** le tensioactif est choisi parmi le laurylsulfate de sodium, les poloxamères et les polysorbates.

7. Composition selon l'une quelconque des revendications 1 à 6, **caractérisée en ce qu'**elle comprend également un agent propénétrant.

8. Utilisation d'une composition selon l'une quelconque des revendications 1 à 7 pour la préparation d'un médicament prévu pour le traitement de :
- symptômes dermatologiques ayant un composant immunoallergique inflammatoire et avec ou sans trouble de la prolifération cellulaire, notamment le psoriasis cutané, muqueux ou unguéal, le rhumatisme psoriasique et l'atopie cutanée telle que l'eczéma.

9. Utilisation selon la revendication 8 pour le traitement du psoriasis.
